Europäisches Patentamt
European Patent Office
Office européen des brevets

(19)

(11) Publication number: **0 132 101**
**A1**

(12) **EUROPEAN PATENT APPLICATION**

(21) Application number: 84304690.5

(22) Date of filing: 10.07.84

(51) Int. Cl.[4]: C 07 D 499/00
A 61 K 31/43
//C07F7/18, C07D233/54

(30) Priority: 14.07.83 US 513569
19.06.84 US 620172

(43) Date of publication of application:
23.01.85 Bulletin 85/4

(84) Designated Contracting States:
AT BE CH DE FR GB IT LI LU NL SE

(71) Applicant: PFIZER INC.
235 East 42nd Street
New York, N.Y. 10017(US)

(72) Inventor: Hamanaka, Ernest Seiichi
40 Eagle Ridge Drive
Gales Ferry Connecticut(US)

(74) Representative: Moore, James William
Pfizer Limited Ramsgate Road
Sandwich Kent CT13 9NJ(GB)

(54) **2-Heterocycloalkylthiopenem derivatives.**

(57) 2-nitrogen heterocycloalkylthio-2-penem-3-carboxylic acid compounds of the formula

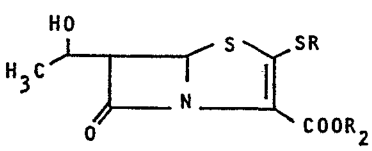

or a pharmaceutically acceptable salt thereof, wherein:

R is

$$-(alk)-X-R_1$$

X is imidazolyl, 1,2,3-triazolyl, 1,2,4-triazolyl or tetrazolyl;

$R_1$ is hydrogen or alkyl having 1-4 carbon atoms;

$R_2$ is hydrogen or a group which forms an ester which is hydrolyzed *in vivo*; and

alk is alkylene having 1-4 carbon atoms.

are useful as antibacterials for treating mammals.

EP 0 132 101 A1

P.C.(Ph) 6726A

## 2-HETEROCYCLOALKYLTHIOPENEM DERIVATIVES

This invention is directed to a family of antibacterial agents incorporating a 2-azetidinone (beta-lactam) ring. Chemically, the antibacterial agents of this invention are identified as 6-alpha-hydroxyethyl-2-substituted-2-penem-3-carboxylic acid compounds.

Although certain 2-substituted-2-penem-3-carboxylic acid compounds have been previously disclosed, there is a continuing need for novel compounds having desirable antibacterial therapeutic properties.

The present invention is directed to a compound of the formula

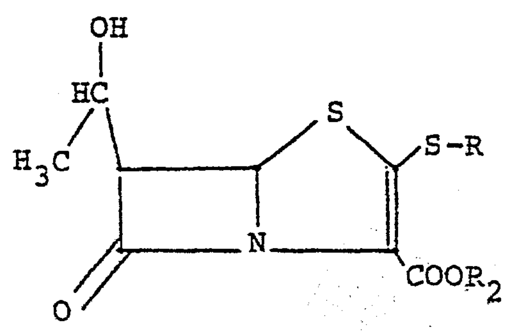

---I

or a pharmaceutically acceptable salt thereof, wherein:

R is

$$-(alk)-X-R_1$$

wherein X is imidazolyl, 1,2,3-triazolyl, 1,2,4-

triazolyl or tetrazolyl; $R_1$ is hydrogen or alkyl having 1-4 carbon atoms, and alk is alkylene having 1-4 carbon atoms, and

$R_2$ is hydrogen or a group which forms an ester which is hydrolyzed in vivo.

Included within the scope of the present invention is a compound of the formula

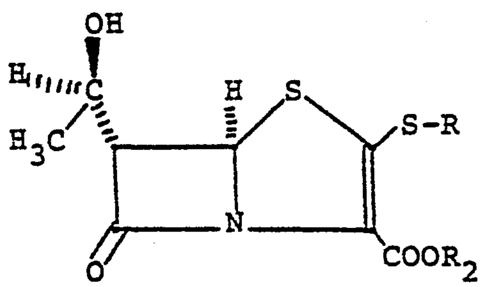

---II

or a pharmaceutically acceptable salt thereof, wherein R and $R_2$ are as defined above for compounds of formula I.

Preferred compounds of formula I and II include those wherein X is imidazolyl, and particularly preferred compounds include those wherein R is 2-(imidazol-1-yl)ethyl, (1-methylimidazol-2-yl)methyl, (imidazol-4-yl)methyl, or 1-(imidazol-1-yl)prop-2-yl.

Further embraced by the present invention is a pharmaceutical composition comprising a compound of formula I or II and a pharmaceutically acceptable diluent or carrier; and a method of treating a bacterial infection in a mammal comprising administering an antibacterially effective amount of a compound of formula I or II.

The compounds of formulas I and II are useful as antibacterial agents, and are derivatives of the bicyclic nucleus of the formula:

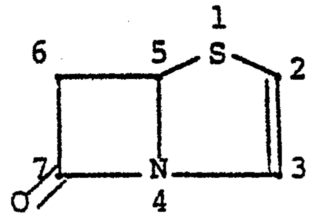

----III

Throughout this specification, the nucleus of formula III is identified by the name "2-penem," and ring atoms are numbered as shown. The carbon atom attached to ring carbon 6 is given the number 8. Also, throughout this specification, the abbreviation "PNB" is used for the p-nitrobenzyl group.

The relationship between the hydrogen on bridgehead carbon 5 and the remaining hydrogen on carbon 6 in compounds of formula I can either be *cis* or *trans*. The present invention embraces both isomers as well as mixtures thereof. The *trans* isomer is generally preferred in pharmaceutical applications and the *cis* isomer can be readily converted to the *trans*-isomer.

Generally, carbon 5 will have the absolute stereochemistry designated *R* using the Prelog-Ingold *R*, *S* sterochemical notation which is employed in this application. Thus, for example, a compound of formula II wherein $R_2$ is hydrogen and R is 1-(imidazol-1-yl)prop-2-yl is named (5*R*, 6*S*)-6-[(*R*)-1-hydroxyethyl]-2-(1-(imidazol-1-yl)prop-2-yl)-thio-3-carboxyl-2-penem.

As will be appreciated, various optically active isomers of the new compounds are possible. The present invention embraces such optically active isomers as well as mixtures thereof.

The present invention is directed to penems substituted in the 2-position by a moiety of the general

formula $-S-(alk)-X-R_1$. It will be appreciated that the heterocyclic groups embraced by X can be linked to alk or $R_1$ at any imino or methylidene positions on the five-membered heterocyclic ring. $R_1$ and (alk) preferably each independently have 1-4 carbon atoms and can be either a straight chain or branched.

The present invention includes those penems in which the 3-carboxyl group is esterified to form a nontoxic ester group which is hydrolyzed *in vivo*, i.e. in mammalian blood or tissue, to release the corresponding penem-3-carboxylic acid or a salt thereof. Typical examples of such readily hydrolyzable ester-forming residues are alkanoyloxymethyl having from 3-8 carbon atoms, 1-(alkanoyloxy)ethyl having from 4-9 carbon atoms, 1-methyl-1-(alkanoyloxy)ethyl having from 5-10 carbon atoms, alkoxycarbonyloxymethyl having from 3-6 carbon atoms, 1-(alkoxycarbonyloxy)ethyl having from 4-7 carbon atoms, 1-methyl-1-(alkoxycarbonyloxy) ethyl having from 5-8 carbon atoms, N-(alkoxycarbonyl) aminomethyl having from 3-9 carbon atoms, 1-(N-[alkoxycarbonyl]amino)ethyl having from 4-10 carbon atoms, 3-phthalidyl, 4-crotonolactonyl, *gamma*-butyrolacton-4-yl, carboxyalkylcarbonyloxymethyl having from 4-12 carbon atoms or 5-methyl-2-oxo-1,3-dioxolen-4-yl-methyl.

To prepare compounds of formula I or II wherein $R_2$ is a group which forms an ester which is hydrolyzed *in vivo*, the acid of formula I or II ($R_2$ is hydrogen) is reacted with a base to form the corresponding anion. Suitable cations include sodium, potassium, calcium, tetra-alkylammonium and the like. The anion can be prepared by lyophilizing an aqueous solution of I or II, for example, an aqueous solution containing tetrahydrofuran,

and sodium bicarbonate or tetrabutylammonium hydroxide.

The resulting anion of I or II is reacted with the corresponding chloride or bromide of $R_2$ in a reaction-inert solvent such as acetone or dimethylformamide at about 20 to about 50°C, preferably 25°C.

The compounds of formula I or II wherein $R_1$ is hydrogen or a salt thereof can be synthesized according to Schemes A-C.

As shown in Scheme A, a compound of formula II can be prepared in accordance with the procedure of Yoshida et al, Chem. Pharm. Bull., 29, 2899-2909(1981), from the known dibromo penam of formula IV. The dibromo penam (V) undergoes an exchange reaction with t-butyl magnesium chloride at a temperature of between about -90 and -40°C, preferably about -76°C in a reaction-inert solvent such as tetrahydrofuran, diethyl ether or toluene, preferably tetrahydrofuran. Other organometallic reagents may also be employed. The resultant reaction mixture is treated *in situ* with the appropriate aldehyde; e.g., acetaldehyde for the 1-hydroxyethyl derivative. The aldehyde is added at between about -80 and -60°C, preferably about -76°C for acetaldehyde.

SCHEME A

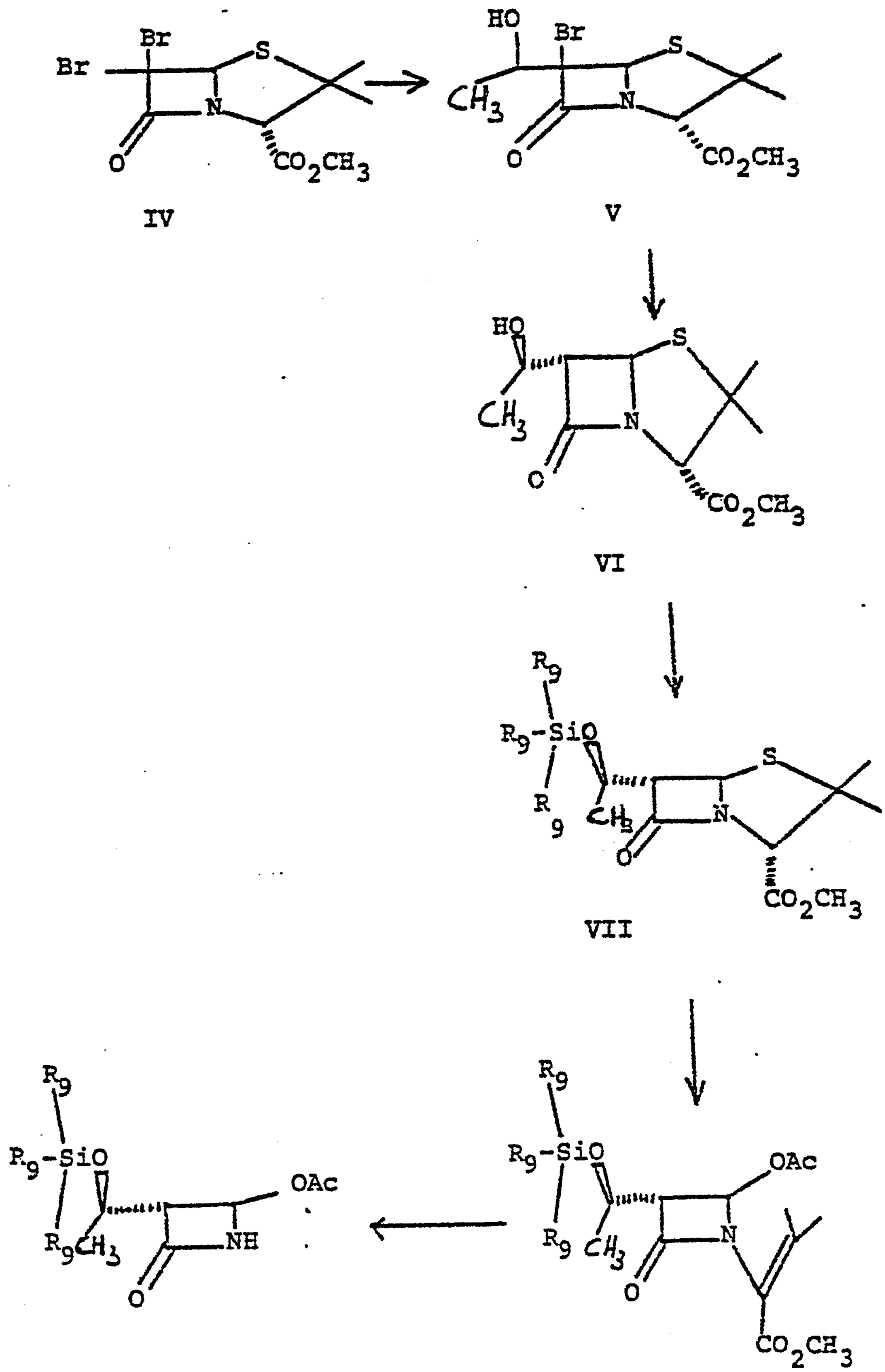

IV

V

VI

VII

VIII

IX

**SCHEME B**

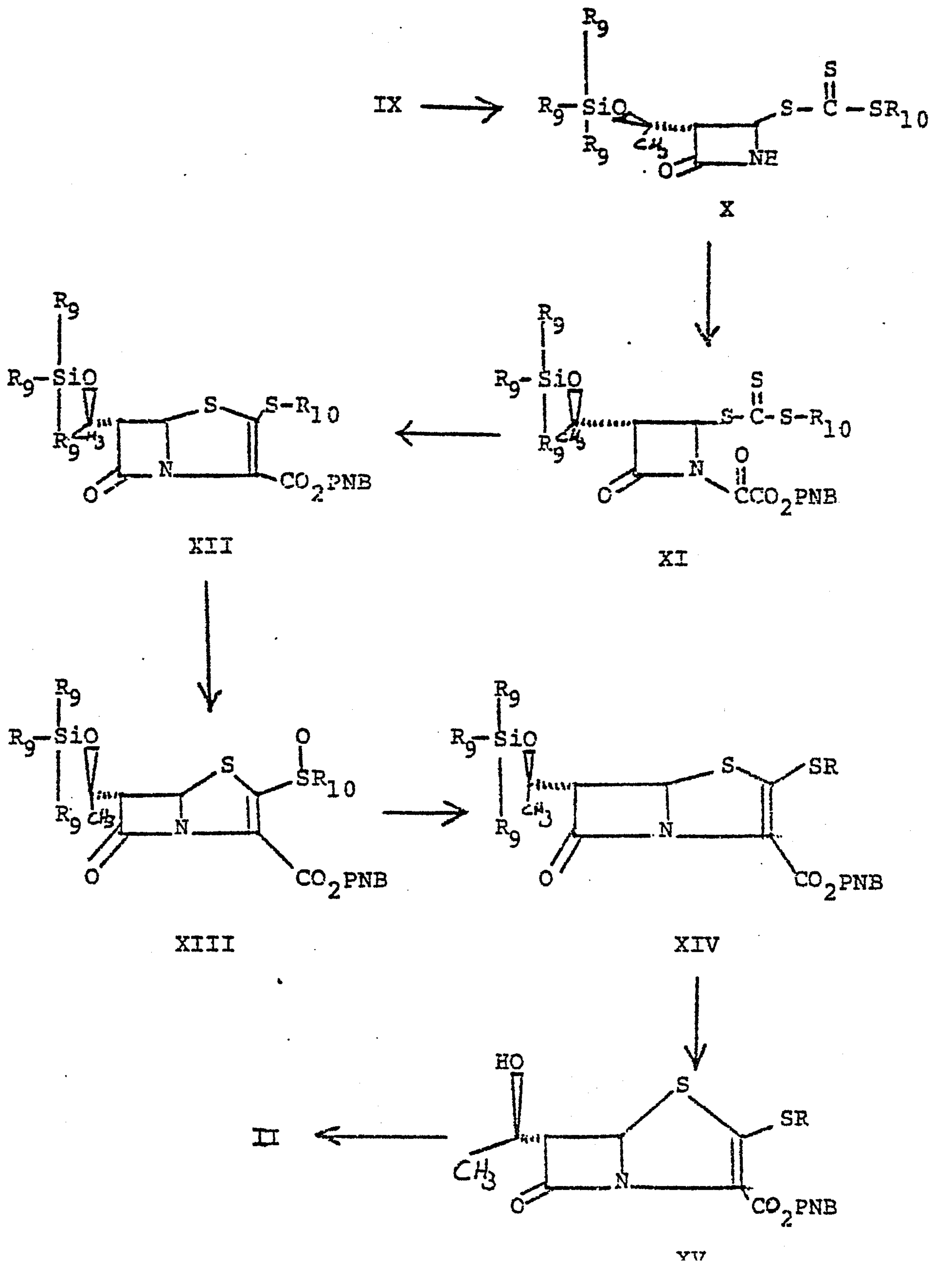

IX

X

XI

XII

XIII

XIV

II

XV

SCHEME C

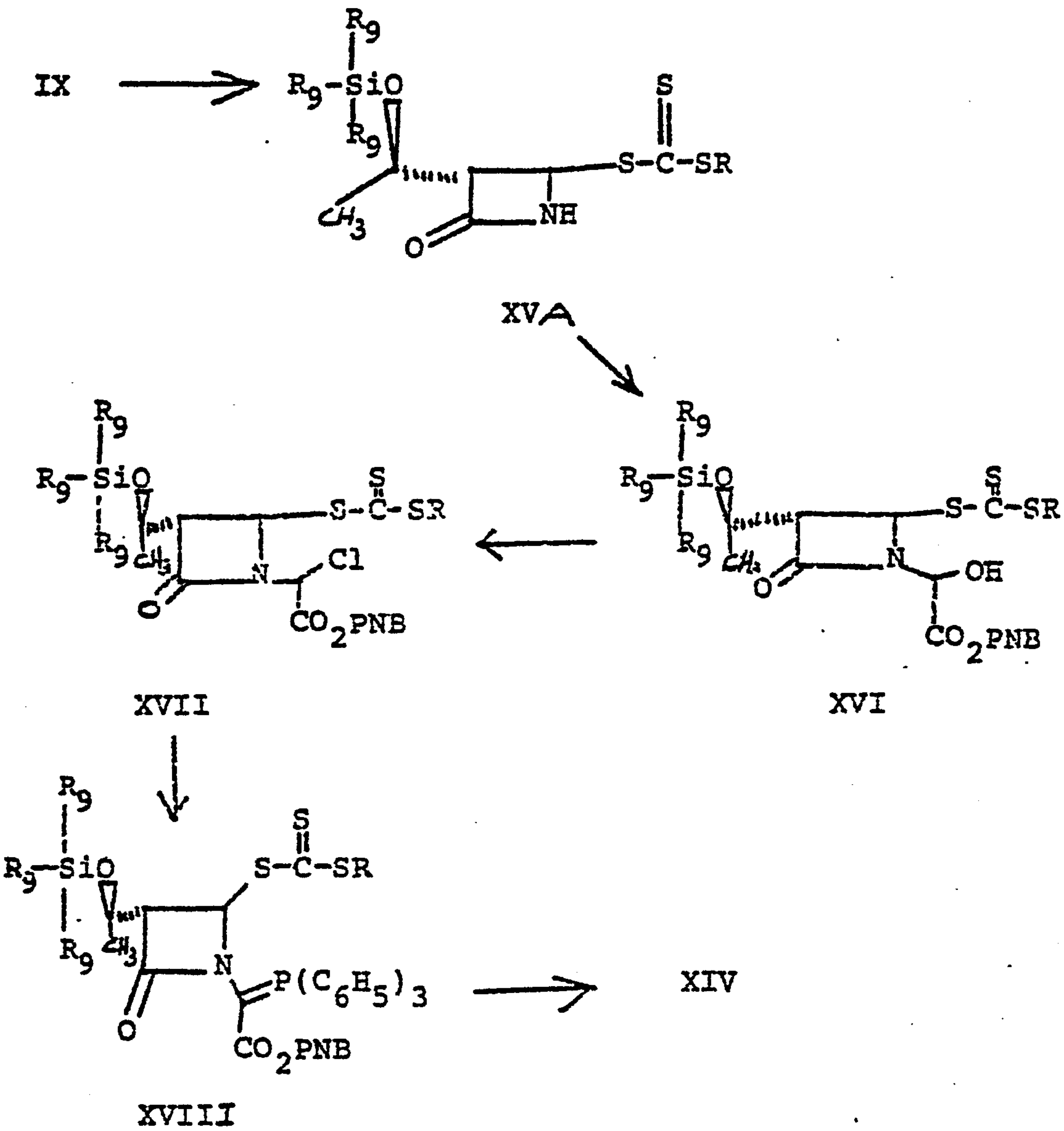

IX

XVA

XVII

XVI

XVIII

XIV

The resulting bromo hydroxy penam V is hydrogenated to remove the 6-bromo substituent. A suitable hydrogenation catalyst is a noble metal catalyst such as palladium. The reaction is carried out in a protic solvent such as 1:1 methanol-water or 1:1 tetrahydrofuran-water, preferably 1:1 methanol-water, at a pressure of about 1 to 4 atms, preferably 4 atm and a temperature of between about 0 and 30°C, preferably about 25°C.

The resulting alcohol of formula VI can be protected with a trialkylhalosilane of the formula

$$R_9-\overset{\displaystyle R_9}{\underset{\displaystyle R_9}{\overset{|}{\underset{|}{Si}}}}-Q$$

wherein $R_9$ at each occurrance is an alkyl of 1 to 6 carbon atoms and Q is chloro, bromo or iodo. Thus, dimethyl-t-butylchlorosilane in the presence of an amine proton acceptor such as imidazole in a polar, aprotic solvent such as N,N-dimethylformamide at a temperature range of between about 5 and 40°C, preferably about 25°C forms a trialkylsilyl hydroxyl-protecting group as shown in formula VII.

Treatment of VII with mercuric acetate in acetic acid at a temperature of about 90°C yields the olefin VIII.

In order to obtain the desired azetidinone IX, the olefin VIII is ozonized in a reaction-inert solvent such as dichloromethane at a temperature of between about -80 and -40, preferably about -76°C. The reaction product is treated with an alkanol such as methanol to yield the azetidinone IX.

As shown in Scheme B, a compound of formula IX is treated with trithiocarbonate salt of the formula $M^{+}R_{10}$-S-C(S)-$S^{-}$ wherein $R_{10}$ is alkyl having 1-4 carbon atoms, preferrably ethyl, and M is a metal such as sodium or potassium to obtain a compound of formula X. This conversion of IX to X is carried out in an organic solvent or water, preferably a mixture of water and dichloromethane at a temperature range of about 0-35°C, preferably about 25°C.

The compound of formula X is condensed with p-nitrobenzyl chloro-oxalate in the presence of a tertiary alkylamine wherein each alkyl has, for example, 1-4 carbon atoms such as ethyl-di-isopropylamine, to obtain the compound of formula XI. This condensation reaction is carried out in a reaction-inert solvent, preferably dichloromethane, at a temperature range of about 5-25 °C, preferably about 10°C.

The resulting compound of formula XI is cyclized using a trialkyl phosphite wherein alkyl has 1-4 carbon atoms such as triethylphosphite in a reaction-inert solvent such as trichloromethane at a temperature range of about 40-80°C, preferably about 60°C, to obtain the penem of formula XII.

The thio group of compound XII is oxidized to the corresponding sulfoxide XIII with an oxidizing agent such as m-chloroperbenzoic acid in a reaction inert solvent such as dichloromethane, at a temperature range of about -10 to -30°C, preferably -20°C.

The sulfoxide XIII is substituted with the mercaptide of formula R-$S^{-}$ by employing, for example, the sodium or potassium salt which is reacted with the sulfoxide XIII in an polar organic solvent such as ethanol or acetonitrile at a temperature range of about 35 to -50°C, preferably about -35°C.

Starting mercaptans of the formula R-SH or starting thioacetates of the formula $R-S-C(O)CH_3$ are known or can be prepared by analogous methods known in the art. For a review see J.L. Wardell, "Preparation of Thiols," in The Chemistry of the Thiol Group, S. Patai, editor, John Wiley & Sons, London, 1974, Chapter 4.

Mercaptans or their derivatives which are required can be prepared from the corresponding alcohols, halides, p-toluenesulfonates, methanesulfonates and the like. Suitable thiol-derivatives which are readily converted to thiols include thioacetates (by saponification) and t-butylthioethers or p-methoxybenzylthioethers (by treatment with acid or mercuric salts).

See also Volante, Tetrahedron Letters, 22, 3119-3122(1981) for the conversion of alcohols to thiols and thiolesters using triphenylphosphine and a dialkyl azodicarboxylate in the presence of the alcohol and an appropriate thiolacid.

For compounds of formula XIV the trialkylsilyl group is preferably removed prior to the hydrogenolysis to remove the acid-protecting group (PNB) to obtain a compound of formula XV. The trialkylsilyl group is removed with a tetraalkylammonium fluoride in an ethereal solvent such as tetrahydrofuran at a temperature range of about 15 to 40°C, preferably about 25°C.

Conversion of a compound of formula XV to a compound of formula I or II is accomplished using a conventional hydrogenolysis reaction, and it is carried out in conventional fashion for this type of transformation. Thus, a solution of a compound of the formula XV is stirred or shaken under an atmosphere of

hydrogen, or hydrogen mixed with an inert diluent such as nitrogen or argon, in the presence of a catalytic amount of a noble metal hydrogenolysis catalyst, such as a palladium-on-calcium carbonate or a palladium-on-Celite (a diatomaceous earth) catalyst. Convenient solvents for this hydrogenolysis are lower alkanols, such as methanol; ethers, such as tetrahydrofuran and dioxan; low molecular weight esters, such as ethyl acetate and butyl acetate; water; and mixtures of these solvents. However, it is usual to choose conditions under which the starting material is soluble. The hydrogenolysis is usually carried out at room temperature and at a pressure from about 0.5 to about 5 $kg/cm^2$. The catalyst is usually present in an amount from about 10 percent by weight based on the starting material up to an amount equal in weight to the starting material, although larger amounts can be used. The reaction commonly takes about one hour, after which the compound of the formula I or II is recovered simply by filtration followed by removal of the solvent in vacuo. If palladium-on-calcium carbonate is used as the catalyst, the product is often isolated as the calcium salt and if palladium-on-Celite (a diatomaceous earth) is employed, the product is often isolated as the sodium salt.

The compound of formula I or II can be purified by conventional methods for beta-lactam compounds. For example, a compound of formula I or II can be purified by column chromatography gel filtration on Sephadex, or by recrystallization.

An alternate synthetic procedure is shown in Scheme C. The azetidinone of formula IX is reacted with a tri-thiocarbonate of the formula $M^{+}R-S-C(S)-S^{-}$, wherein M is a metal such a sodium or potassium, using the procedure previously described to prepare X.

The resulting trithiocarbonate XVA is treated with (p-nitrobenzyloxycarbonyl)(dihydroxy)methane in an aprotic solvent such as benzene, toluene or dimethylformamide, preferably benzene, at a temperature range of about 25-110°C, preferably about 80°C to yield the alcohol of formula XVI.

The corresponding chloride XVII is prepared from the alcohol XVI by treatment with thionyl chloride in a reaction-inert organic solvent such as dichloromethane in the presence of a hindered amine which serves as an acid acceptor such as 2,6-lutidine at a temperature range of about -10 to 75°C, preferably 0°C.

The chloride XVII is reacted with a triarylphosphine such as triphenylphosphine in a reaction-inert solvent such as tetrahydrofuran in the presence of a tertiary amine such as 2,6-lutidine at a temperature of about 25°C, to obtain the compound of formula XVIII which is cyclized by refluxing in an aromatic solvent such as toluene to yield the penem of formula XIV.

Trithiocarbonate salts of the formula $M^{+}R\text{-}S\text{-}(C{=}S)\text{-}S^{-}$ are prepared from the appropriate mercaptan of the formula R-SH or by treatment of a thioacetate of the formula $RSC(O)CH_3$ with an alkali metal alkoxide followed by carbon disulfide.

By employing the heretofore mentioned procedure of Yoshida et al., the stereochemistry at carbon 6 of the penem as well as the hydroxyethyl group attached to carbon 6 is that shown in formula II. The principal stereochemistry of the product of ring closure using Schemes B or C is that wherein the hydrogen at penem ring position 5 is <u>trans</u> to the hydrogen on carbon 6 and in the <u>alpha</u> configuration.

The compounds of formula I or II are acidic and will form salts with basic agents. Such salts are considered to be within the scope of this invention. These salts can be prepared by standard techniques, such as contacting the acidic and basic components, usually in a stoichiometric ratio, in an aqueous, non-aqueous or partially aqueous medium, as appropriate. They are then recovered by filtration, by precipitation with a nonsolvent followed by filtration, by evaporation of the solvent, or in the case of aqueous solutions by lyophilization, as appropriate. Basic agents which are suitably employed in salt formation belong to both the organic and inorganic types, and they include ammonia, organic amines, alkali metal hydroxides, carbonates, bicarbonates, hydrides and alkoxides, as well as alkaline earth metal hydroxides, carbonates, hydrides and alkoxides. Representative examples of such bases are primary amines, such as n-propylamine, n-butylamine, aniline, cyclohexylamine, benzylamine and octylamine; secondary amines, such as diethylamine, morpholine, pyrrolidine and piperidine; tertiary amines, such as triethylamine, N-ethylpiperidine, N-methylmorpholine and 1,5-diazabicyclo-[4,3,0]non-5-ene; hydroxides, such as sodium hydroxide, potassium hydroxide, ammonium hydroxide and barium hydroxide; alkoxides, such as sodium ethoxide and potassium ethoxide; hydrides, such as calcium hydride and sodium hydride; carbonates, such as potassium carbonate and sodium carbonate; bicarbonates, such as sodium bicarbonate and potassium bicarbonate; and alkali metal salts of long-chain fatty acids, such as sodium 2-ethylhexanoate.

Preferred salts of the compounds of formula I or II are sodium, potassium and calcium salts.

The pharmaceutically acceptable salts of formula I or II are those which are free of significant adverse side-effects at the level of ordinary use and include, e.g., the sodium, potassium or calcium salts thereof.

The *in vitro* antibacterial activity of the compounds of the formula I or II and salts thereof can be demonstrated by measuring their minimum inhibitory concentrations (MIC's) in mcg/ml against a variety of microorganisms. The procedure which is followed is the one recommended by the International Collaborative Study on Antibiotic Sensitivity Testing (Ericcson and Sherris, *Acta. Pathologica et Microbiologia* Scandinav, Supp. 217, Section B: 6468 [1971]), and employs brain heart infusion (BHI) agar and the inocula replicating device. Overnight growth tubes are diluted 100 fold for use as the standard inoculum (20,000-10,000 cells in approximately 0.002 ml. are placed on the agar surface; 20 ml. of BHI agar/dish). Twelve 2 fold dilutions of the test compound are employed, with initial concentration of the test drug being 200 mcg/ml. Single colonies are disregarded when reading plates after 18 hrs. at 37°C. The susceptibility (MIC) of the test organism is the lowest concentration of compound capable of producing complete inhibition of growth as judged by the naked eye.

The compounds of formula I or II, and the pharmaceutically-acceptable salts thereof, are suitable for the control of bacterial infections by susceptible bacteria in mammals, including man, e.g. infections caused by susceptible strains of *Staphylococcus aureus*.

The compounds of the present invention can be administered orally or parenterally, i.e. intramuscularly, subcutaneously, intraperitoneally or intravenously, alone, or combined with a pharmaceutically-acceptable carrier. The ratio of active ingredient to carrier will depend on the chemical nature, solubility and stability of the active ingredient, as well as the dosage contemplated. The ratio of the pharmaceutically-acceptable carrier to the penem compound will normally be in the range from about 1:10 to 4:1. For oral administration, the compounds of this invention can be used in the form of tablets, capsules, lozenges, troches, powders, syrups, elixirs, aqueous solutions and suspensions, and the like. In the case of tablets, carriers which can be used include lactose, sodium citrate and salts of phosphoric acid. Various disintegrants such as starch, and lubricating agents, such as magnesium stearate, sodium lauryl sulfate and talc, are commonly used in tablets. Useful diluents for capsules include lactose and high molecular weight polyethylene glycols. When aqueous suspensions are required for oral use, the active ingredient may be combined with emulsifying and suspending agents. Sweetening and/or flavoring agents can be added. For parenteral administration, sterile solutions of the active ingredient are usually prepared, and the pH of the solutions are suitably adjusted and buffered. For intravenous use, the total concentration of solutes should be controlled to render the preparation isotonic.

The prescribing physician will determine the appropriate dose for a given human subject, and this can vary according to the age, weight, and response of the individual patient, as well as the nature and the severity of the symptoms. The compounds of formula I or II will normally be used orally at dosages in the range from about 10 to about 200 mg. per kilogram of body weight per day, and parenterally at dosages from about 10 to about 400 mg. per kilogram of body weight per day. In some cases it may be necessary to use dosages outside these limits.

The following Examples and Preparations are provided solely for further illustration. Infrared (IR) spectra were measured either as potassium bromide discs (KBr disc), Nujol mulls (Nujol) or as solutions in chloroform ($CHCl_3$), methylene chloride ($CH_2Cl_2$) or dimethyl sulfoxide (DMSO), and diagnostic absorption bands are reported in either microns or wave numbers ($cm^{-1}$). Nuclear magnetic resonance (NMR) spectra were measured for solutions in deuterochloroform ($CDCl_3$) or perdeuterodimethyl sulfoxide (DMSO-$d_6$), or mixtures thereof, and peak positions are expressed in parts per million downfield from tetramethylsilane. The following abbreviations for peak shapes are used: s, singlet; d, doublet; t, triplet; q, quartet; m, multiplet; b, broad; c, complex. The abbreviations "ss" and "sss" denote that a particular proton appeared as two or three singlets respectively, owing to the presence of diastereoisomers. Throughout the Examples and Preparations, the abbreviation "PNB" represents the p-nitrobenzyl group.

EXAMPLE 1

Sodium (5R, 6S)-6-[(R)-1-Hydroxyethyl]-2-[(imidazol-1-yl)-ethylthio]-2-penem-3-carboxylate

A suspension of 38 mg 10% palladium on diatomaceous earth in 10 ml. tetrahydrofuran and 10 ml. water was adjusted to a pH of 7.0 with 0.01N aqueous hydrochloric acid solution. A solution of 38 mg p-nitrobenzyl (5R, 6S)-6-[(R)-1-hydroxyethyl]-2-(imidazol-1-yl)ethylthio-2-penem-3-carboxylate in 5 ml. tetrahydrofuran and 5 ml. distilled water was added and the resulting mixture was hydrogenated at 55 p.s.i. of hydrogen for 75 min.; 38 mg of 10% palladium on diatomaceous earth was added to the reaction mixture and the pH of the suspension was adjusted to 7.0 with 0.02M aqueous sodium bicarbonate solution. The mixture was hydrogenated at 55 p.s.i. of hydrogen for 75 min., then the catalyst was removed by filtration and the filtrate was concentrated in vacuo to remove tetrahydrofuran. The pH of the resulting aqueous solution was adjusted to 7.0 and the solution was extracted with 25 ml. ethyl acetate. The aqueous layer was then lyophilized, yielding 23 mg (80% yield) of the title compound as an amorphous solid.

The infrared spectrum of the title compound as a potassium bromide disc had absorptions at 2.93, 5.65 and 6.28 microns.

The 250 MHZ NMR spectrum of a perdeuterodimethylsulfoxide solution of the title compound has peaks at 1.16(d,3H); 3.2(m,2H); 3.54(m,1H); 3.92(m,1H); 4.18 (m,2H); 5.54(s,1H); 6.88(s,1H); 7.2(s,1H); and 7.65 (s,1H) ppm.

## EXAMPLE 2

Sodium (5R, 6S)-6-[(R)-1-Hydroxyethyl]-2-(imidazol-4-yl)methylthio-2-penem-3-carboxylate

The procedures of Example I were employed using p-nitrobenzyl sodium (5R, 6S)-6-[(R)-1-6-alpha-hydroxyethyl]-2-(imidazol-4-yl)methylthio-2-penem-3-carboxylate as the starting material to obtain sodium (5R, 6S)-6-[(R)-1-Hydroxyethyl]-2-(4-imidazolylmethylthio 2-penem-3-carboxylate in 38% yield. The infrared spectrum of the product as a potassium bromide disc had absorptions at 2.93, 5.65 and 6.26 microns.

Using as the starting material p-nitrobenzyl (5R, 6S)-6-[(R)-1-hydroxyethyl]-2-[1-(imidazol-1-yl)prop-2-yl]thio-2-pene 3-carboxylate derived from the less polar diastereomer the corresponding sodium salt of the product of formula II was obtained in 97.5% yield (IR (Nujol mull): 5.65 microns).

Similarly, using as the starting material the compound of formula XV wherein R is 1-(imidazol-1-yl)prop-2-yl derived from the more polar diastereomer the corresponding sodium salt of the product of formula II was obtained in 60% yield (IR (Nujol mull): 5.66 microns).

## EXAMPLE 3

Calcium (5R, 6S)-6-[(R)-1-Hydroxyethyl]-2-(1-methylimidazol-2-yl)methylthio-2-penem-3-carboxylate

A solution of 0.12 g of p-nitrobenzyl (5R, 6S)-6-[(R)-1-hydroxyethyl]-2-(1-methylimidazol-2-yl)methylthio-2-penem-3-carboxylate in 15 ml. tetrahydrofuran and 5 ml. water was added to a suspension of 0.12 g of 5% palladium-on-calcium carbonate in 20 ml. water and 10 ml. tetrahydrofuran that had been prehydrogenated at 55 p.s.i. of hydrogen. The mixture was hydrogenated at 55 p.s.i. of hydrogen for 45 min., then 0.12 g of

5% palladium-on-calcium carbonate was added and the mixture was hydrogenated at 55 p.s.i. for a further 60 min. The catalyst was removed by filtration and the filtrate was concentrated *in vacuo* to remove tetrahydrofuran. The aqueous solution was washed with three 25 ml. portions of ethyl acetate, then lyophilized, yielding 74 mg. (82% yield) of the title compound as an amorphous solid.

The infrared spectrum of the title compound as a potassium bromide disc had absorptions at 2.92, 5.7 and 6.28 microns.

The 250 MHZ NMR spectrum of perdeuterodimethylsulfoxide solution of the title compounds has peaks at 1.16(d,3H); 3.55-3.7(m with s at 3.64, total 4H); 3.94 (m,1H); 4.21(q,2H); 5.56(s,1H); 6.8(s,1H); and 7.1 (s,1H) ppm.

PREPARATION A

p-Nitrobenzyl (5R, 6S)-6-[(R)-1-Hydroxyethyl]-2-(imidazol-1-yl)ethylthio-2-penem-3-carboxylate

To a solution of 152 mg (0.258 mmole) *p*-nitrobenzyl (5*R*, 6*S*)-6-[(*R*)-1-*t*-butyldimethylsilyloxyethyl]-2-[(imidazol-1-yl)ethylthio]-2-penem-3-carboxylate in 7 ml. tetrahydrofuran was added 0.15 ml (2.58 mmoles) acetic acid and 0.774 ml. (0.774 mmole) of a 1M solution of tetrabutylammonium fluoride in tetrahydrofuran. After stirring for 24 hr. under nitrogen at room temperature, 50 ml. ethyl acetate was added and the resulting solution was washed with 30 ml. saturated aqueous sodium bicarbonate solution, 30 ml. water, and 30 ml. saturated aqueous sodium chloride solution. The ethyl acetate layer was dried over anhydrous sodium sulfate and concentrated *in vacuo*. The crude product (152 mg.) was purified by column chromatography on silica gel (50 g.), eluting with 85:15 ethyl acetate-methanol, to yield 77 mg. (63% yield) of the title compound as an amorphous solid.

The infrared spectrum of a dichloromethane solution of the title compound showed absorptions at 5.57, 5.92 and 6.58 microns.

The NMR spectrum of a deuterochloroform solution of the title compound showed peaks at 1.34(d,3H); 2.76-3.38 (c,2H); 3.72(m,1H); 4.24(m,3H); 5.28(q,2H); 5.62(d,1H); 6.93(m,2H); 7.5(d,3H); and 8.13(d,2H) ppm.

## PREPARATION B

The procedures of Preparation A were employed to obtain the compound of formula XV, wherein R and the physical data are as shown in Table I, from the corresponding compound of formula XIV. The solvents are shown in parenthesis.

Table I

| R | I.R. (microns) | NMR(ppm) | Yield (%) |
|---|---|---|---|
| (imidazol-4-yl)-methyl | 5.57,5.92 and 6.56 ($CH_2Cl_2$) | 1.28 (d, 3H); 3.68 (m,1H); 4.2(c, 3H); 5.27 (q, 2H); 5.6(d, 1H); 6.98 (s, 1H); 7.6 (m, 3H); and 8.2 (d, 2H) ($CDCl_3$) | 36 |

Table I continued

| R | I.R. (microns) | NMR(ppm) | Yield (%) |
|---|---|---|---|
| (1-methyl-imidazol-2-yl)-methyl | 5.6 and 5.92 (Nujol) | 1.17 (d, 3H); 3.64(s, 3H); 3.87 (m, 1H); 4.0(m, 1H); 4.42(q, 2H); 5.24 (d, 1H); 5.37 (q, 2H); 5.75(d, 1H); 6.83(d, 1H); 7.14(d, 1H); 7.7 (d, 2H); and 8.24 (d, 2H). (250 MHZ, DMSO-$d_6$) | 71 |
| 1-(imidazol-1-yl)-prop-2-yl (from less polar diastereomer) | 5.58,5.89 and 6.59 ($CH_2Cl_2$) | 1.37 (d, 6H); 3.3-4.5 (c, 6H); 5.3 (q, 2H); 5.58 (d, 1H); 6.86 (m, 1H); 6.98 (m, 1H); 7.44 (m) and 7.52 (d) (total 3H); and 8.16 (d, 2H). ($CDCl_3$) | 63.5 |

Table I continued

| R | I.R. (microns) | NMR(ppm) | Yield (%) |
|---|---|---|---|
| 1-(imidazol-1-yl)-prop-2-yl (from more polar diastereomer) | 5.59, 5.9 and 6.59 ($CH_2Cl_2$) | 1.36 (d, 6H); 3.28-4.45 (c, 6H); 5.28 (q, 2H); 5.62 (d, 1H); 6.86 (m, 1H); 6.96 (m, 1H); 7.42 (m) and 7.52 (d) (total 3H); and 8.12 (d, 2H) ($CDCl_3$) | 39.8 |

PREPARATION C

p-Nitrobenzyl (5R, 6S)-6-[(R)-1-t-Butyldimethylsiloxyethyl]-2-((imidazol-1-yl)ethylthio)-2-penem-3-carboxylate

Sodium methoxide (228 mg., 4.22 mmole) was added to a solution of 715 mg. (4.22 mmole) 2-(imidazolyl)ethyl thioacetate in 35 ml. anhydrous ethanol cooled to -35°C under nitrogen. After 65 min. at -35°C, a solution of 2.5 g.(*ca.* 4.22 mmole) of crude p-nitrobenzyl (5R, 6S)-6-[(R)-1-t-butyldimethylsilyoxyethyl]-2-ethylsulfinyl-2-penem-3-carboxylate in 35 ml. tetrahydrofuran which had been cooled to -50°C was added. The resulting solution was stirred at -35°C to -40°C for 75 min., then 0.24 ml. (4.22 mmole) acetic acid was added and the solution was concentrated *in vacuo*. The residue was dissolved in 150 ml. ethyl acetate and the resulting solution was

washed with 75 ml. saturated aqueous sodium bicarbonate solution, 75 ml. water and 75 ml. saturated sodium chloride solution. The ethyl acetate layer was dried over anhydrous sodium sulfate and concentrated in vacuo. Chromatography of the crude product (3.08g.) on silica gel (500 g.) eluting with 90:10 ethyl acetate-methanol yielded 980 mg. (39%) of the title compound as a viscous gum.

The IR spectrum of the title compound in dichloromethane had absorptions at 5.58, 5.92 and 6.57 microns.

The NMR spectrum of a deuterochloroform solution of the title compound had peaks at 0.03(s, 3H); 0.08 (s, 3H); 0.8 (s, 9H); 1.23 (d, 3H); 2.75 - 3.4(c, 2H); 3.72(m, 1H); 4.28(m, 3H); 5.3(q, 2H); 5.66(d, 1H); 7.0 (m, 2H); 7.6 (d, 3H); and 8.2 (d, 2H) ppm.

## PREPARATION D

## p-Nitrobenzyl (5R, 6S)-6-[(R)-1-t-Butyldimethylsiloxyethyl]-2-(imidazol-4-yl)methylthio-2-penem-3-carboxylate

The procedures of Preparation C were employed substituting imidazol-4-ylmethyl thioacetate as the thioacetate to obtain the title compound in 15% yield.

The I.R. spectrum of a dichloromethane solution of the title compound had absorptions at 5.58, 5.92 and 6.57 microns.

The NMR spectrum of a deuterochloroform solution of the title compound had peaks at 0.02(s,3H);0.06 (s,3H); 0.8 (s, 9H); 1.22(d, 3H); 3.66(m, 1H); 4.2 (c, 3H); 5.24 (q, 2H); 5.6(d, 1H); 6.97 (s, 1H); 7.4 (d) and 7.57 (s) (total 3H); and 8.18 (d, 2H)ppm.

PREPARATION E

p-Nitrobenzyl (5R, 6S)-6-[(R)-1-t-Butyldimethylsiloxyethyl-2-(1-methylimidazol-2-yl)methylthio-2-penem-3-carboxylate

The procedures of Preparation C were employed substituting sodium ethoxide for sodium methoxide and employing 1-methylimidazol-2-ylmethyl thioacetate as the thioacetate to obtain the title compound in 44% yield.

The IR spectrum of a Nujol mull of the title compound had absorptions at 5.58 and 5.9 microns.

The NMR spectrum of a deuterochloroform solution of the title compound had peaks at 0.03(s,3H);0.06 (s,3H); 0.84(s, 9H); 1.24(d, 3H); 3.56-3.8 (m with s at 3.7, total 4 H); 3.95 - 4.44(m with s at 4.3, total 3 H); 5.24 (q, 2H); 5.64 (d, 1H); 6.8(d, 1H); 6.9(d, 1H); 7.5 (d, 2H); 8.1 (d, 2H).

PREPARATION F

p-Nitrobenzyl (5R, 6S)-6-[(R)-1-t-Butyldimethylsilyloxy - ethyl]-2-ethylsulfinyl-2-penem-3-carboxylate

A solution of 970 mg. (4.78 mmoles, 85% purity) m-chloroperbenzoic acid in 25 ml. methylene chloride was added to a solution of 2.5 g. (4.78 mmoles) of p-nitrobenzyl (5R, 6S)-6-[(R)-1-t-butyldimethylsilyloxyethyl]-2-ethylthio-2-penem-3-carboxylate in 125 ml. methylene chloride cooled to -20°C under a nitrogen atmosphere. The mixture was stirred at -20°C for 3 hr., then washed sequentially with two 70 ml. portions of saturated aqueous sodium bicarbonate solution, 70 ml. water and 70 ml. saturated aqueous

sodium chloride solution. The methylene chloride solution was dried with anhydrous sodium sulfate and concentrated in vacuo to a yellow foam of the title compound (2.2 g., 86% yield).

The infrared spectrum of the title compound as a dichloromethane solution had absorptions at 5.54, 5.86 and 6.53 microns. The NMR spectrum of the title compound as a deuterochloroform solution had peaks at 0.06, 0.08, 0.1 and 0.12 (4s, total 6H); 0.8(s, 9H); 1.12-1.58 (m, 6H); 3.1 (m, 2H); 3.86(m, 1H), 4.3(m, 1H), 5.3 (m, 2H); 5.67 and 5.78 (2d, total 1H); 7.54(d, 2H); and 8.18 (d, 2H)ppm.

PREPARATION G

p-Nitrobenzyl (5R), 6S)-6-[(R)-1-t-Butyldimethylsilyloxyethy 2-ethylthio-2-penem-3-carboxylate

p-Nitrobenzyl oxalyl chloride (5.85 g. 0.024 mole) was added to a mixture of 7.3 g (0.02 mole) (5R, 6S)-6-[(R)-1-t-butyldimethylsilyloxyethyl]-4-ethylthio(thiocarbonyl)thio-2-oxo-azetidine and 4.8 g. (0.048 mole) calcium carbonate in 70 ml. methylene chloride cooled to 10°C under a nitrogen atmosphere. A solution of 4.17 ml. (0.024 mole) diisopropylethylamine in 20 ml. methylene chloride was added dropwise at a rate to keep the temperature below 12°C. The mixture was stirred for 60 min. at 10°C, then washed with two 50 ml. portions of ice cold water, dried over anhydrous sodium sulfate and concentrated in vacuo to a viscous oil. The resulting crude p-nitrobenzyl (3-alpha-(R)-t-butyldimethylsilyloxyethyl-2-oxo-azetidinyl)oxoacetate was dissolved in 300 ml. ethanol-free chloroform and the resulting solution was refluxed under nitrogen while a solution of 6.85 ml. (0.04 mole) triethylphosphite in 50 ml. ethanol-free chloroform was added dropwise

over 2 hr. The resulting solution was refluxed for 16 hr., then concentrated *in vacuo*. The residue was chromatographed on silica gel (800 g.), eluting with 95:5 toluene-ethyl acetate to yield 5.5 g. (53% yield) of the title compound as a yellow foam.

The infrared spectrum of the title compound as a dichloromethane solution had absorptions at 5.56, 5.89 and 6.54 microns. The NMR spectrum of the title compound as a deuterochloroform solution had peaks at 0.07(s,3H); 0.1(s,3H); 0.85(s,9H); 1.12-1.53(m,6H); 2.97(q,2H); 3.7(m,1H); 4.25(m,1H); 5.3(q,2H); 5.63 (d,1H); 7.38(d,2H); and 8.18(d,2H)ppm.

The NMR spectrum of the intermediate 4-ethylthio (thiocarbonyl)thio-azetidinone as a deuterochloroform solution had peaks at 0.06(s,6H); 0.8(s,9H); 1.14-1.62(m,6H); 3.14-3.63(m,3H); 4.33(m,1H); 5.16(s,2H); 6.7(d,1H); 7.5(d,2H); and 8.17(d,2H)ppm.

## PREPARATION H

### 3-*alpha*-(*R*)-*t*-Butyldimethylsilyloxyethyl-4-ethylthio (thiocarbonyl)thio-2-oxo-azetidine

Ethanethiol (8.5 ml. 0.115 mole) was added to a solution of 4.18 g. (0.104 mole) sodium hydroxide in 250 ml. water cooled to 0-5°C under a nitrogen atmosphere. After 15 min. 7.73 ml. (0.12 mole) carbon disulfide was added and the mixture was stirred at 0-5°C for 35 min. A solution of 15.0 g. (0.0522 mole) 4-acetoxy-3-*t*-butyldimethylsilyloxyethyl-2-azetidinone in 500 ml. methylene chloride was added and the mixture was stirred vigorously at room temperature for 24 hr. The aqueous phase was separated and extracted with two 150 ml. portions of methylene chloride. The

combined methylene chloride fractions were washed with two 200 ml. portions of water and 200 ml. saturated aqueous sodium chloride solution, dried over anhydrous sodium sulfate and concentrated *in vacuo*. The crude title product (18 g.) was chromatographed on silica gel (500 g.), eluting with 99:1 chloroform-ethyl acetate to yield 9.1 g. (48% yield) of title trithiocarbonate as a yellow foam.

The infrared spectrum of the title compound in dichloromethane solution had absorptions at 5.62 and 9.2 microns. The NMR spectrum of a deuterochloroform solution of the title compound had peaks at 0.08(s,6H); 0.8(s,9H); 1.02-1.5(m,6H); 3.0-3.48(m,3H); 4.12(m,1H); 5.54(d,1H); and 6.57(b,1H)ppm.

## PREPARATION I

## 2-(Imidazol-1-yl)ethyl Thioacetate

A mixture of 4.0g (0.015 mole) 2-(imidazol-1-yl)ethyl tosylate and 2.6g (0.0225 mole) potassium thioacetate in 100 ml. acetone was refluxed under nitrogen overnight. The reaction mixture was then filtered and the filtrate was concentrated *in vacuo*. The residue was dissolved in 100 ml. ethyl acetate and 100 ml. water and the ethyl acetate layer was washed with 100 ml. water and 100 ml. saturated aqueous sodium chloride solution, dried over anhydrous sodium sulfate and concentrated *in vacuo*. The crude product was chromatographed on silica gel, eluting with 9:1 ethyl acetate-methanol, to yield 1.0g (39% yield) of the title thioacetate as a yellowish oil.

The NMR spectrum of a deuterochloroform solution of the title compound showed peaks at 2.36 (s, 3H); 3.17 (t, 2H); 4.08 (t, 2H); 6.9 (d, 1H); 6.98(d, 1H); and 7.47(s, 1H)ppm.

PREPARATION J

2-(Imidazol-1-yl)ethyl Tosylate

A solution of 2.24 g. (0.02 mole) 2-(imidazol-1-yl)-ethanol and 4.88g (0.04 mole) 4-dimethylaminopyridine in 100 ml. methylene chloride was cooled to 0°C under nitrogen and 3.8g (0.02 mole) p-toluenesulfonyl chloride was added. The resulting solution was stirred at 0°C for 3 hr., then at room temperature overnight. 100 ml water was added and the pH of the mixture was adjusted to 7.0 with 1N aqueous hydrochloric acid solution. The methylene chloride layer was separated, washed with 70 ml. saturated aqueous sodium chloride solution, dried over anhydrous sodium sulfate and concentrated in vacuo to an oil (4.2g) of the title compound.

The NMR spectrum of a deuterochloroform solution of the title compound had peaks at 2.42 (s, 3H); 4.23 (s, 4H); 6.86 (d, 1H); 7.0 (d, 1H); and 7.16-7.8 (m, 5H) ppm.

PREPARATION K

Imidazol-4-ylmethyl Thioacetate

To a solution of 890 mg (7.8 mmoles) potassium thioacetate in 30 ml. ethanol was added 800 mg (5.2 mmoles) of 4-(chloromethyl)imidazole hydrochloride. The resulting mixture was stirred overnight at room temperature under nitrogen. The reaction mixture was then concentrated to dryness in vacuo. 100 ml. ethyl acetate and 100 ml. saturated aqueous sodium bicarbonate solution were added to the residue. The ethyl acetate layer was separated and washed successively with 70

ml. saturated aqueous sodium bicarbonate solution, 70 ml. water and 70 ml. saturated aqueous sodium chloride solution, dried over anhydrous sodium sulfate and concentrated <u>in vacuo</u>. The crude product was chromatographed on silica gel (150g), eluting with 9:1 ethyl acetate/methanol to yield 300 mg. (37%) of the title thioacetate as a yellowish oil.

The NMR spectrum of a deuterochloroform solution of the title compound had peaks at 2.32 (s, 3H); 4.14 (s, 2H); 6.99 (s, 1H); and 7.64 (s, 1H)ppm.

<u>PREPARATION L</u>

<u>(1-Methylimidazol-2-yl)methyl Thioacetate</u>

A solution of 1.0 g (0.0089 mole) of (1-methylimidazol-2-yl)methanol, 4.7 ml. carbon tetrachloride and 2.93 g. (0.011 mole) triphenylphosphine in 35 ml. chloroform was refluxed under nitrogen for 2 hours. The reaction mixture was then cooled to 25°C and 2.8 g. (0.0089 mole) of tetrabutylammonium thioacetate was added and the resulting solution was stirred at room temperature overnight. The entire reaction mixture was placed on a silica gel column (200 g.). Elution with 2:1 methylene chloride-acetone yielded 600 mg. of product contaminated by an impurity which was removed by repeated column chromatography on silica gel (200 g). Elution with 4:1 methylene chloride-acetone yielded 0.3 g (20 % yield) of the title thioacetate as a yellowish oil.

The NMR spectrum of a deuterochloroform solution of the title compound had peaks at 2.37 (s, 3H); 3.58 (s, 3H); 4.18(s, 2H); 6.72 (d, 1H); and 6.81 (d, 1H)ppm.

PREPARATION M

p-Nitrobenzyl (5R, 6S)-6-[(R)-1-t-Butyldimethylsiloxyethyl]-2-(1-(imidazol-1-yl)prop-2-yl)thio-2-penem-3-carboxylate

Sodium methoxide (36.5 mg, 0.68 mmole) was added to a solution of (2-(methylcarbonylthio)propyl)imidazole (130 mg, 0.71 mmole) in 7 ml. anhydrous ethanol which had been cooled to -35°C under nitrogen. The resulting solution was stirred at -25°C for 1 hr. then cooled to -40°C.

A solution of p-nitrobenzyl (5R, 6S)-6-[(R)-1-t-butyldimethylsilyloxyethyl]-2-ethylsulfinyl-2-penem-3-carboxylate in 7 ml. anhydrous tetrahydrofuran which had been cooled to -50°C was added and the resulting solution was stirred at -40°C for 50 min. Acetic acid (0.68 mmole) was added and the solution was concentrated *in vacuo*. The residue was dissolved in ethyl acetate (40 ml.) and the resulting solution was washed with 30 ml. aqueous sodium bicarbonate solution, 30 ml. water and 30 ml. saturated aqueous sodium chloride solution. The ethyl acetate solution was then dried over anhydrous sodium sulfate and chromatographed on silica gel (150 g), eluting with 95:5 ethyl acetate-methanol to yield 72 mg (18.5% yield) of the less polar diastereomer, 124 mg (31% yield) of a mixture of diastereomers and 72 mg. (18.5% yield) of the more polar diastereomer.

less polar diastereomer

IR($CH_2Cl_2$): 5.57, 5.88, and 6.54 microns.

NMR($CDCl_3$): 0.04 (s, 3H), 0.06 (S, 3H); 0.82 (s, 9H) 1.23 (d, 3H); 1.3 (d, 3H); 3.37-4.46 (c, 5H); 5.25 (q, 2H); 5.56 (d, 1H); 6.86 (m, 1H); 6.98 (m, 1H); 7.42 (m, 1H); 7.54 (d, 2H); and 8.12 (d, 2H) ppm.

more polar diastereomer

IR($CH_2Cl_2$): 5.58, 5.92, and 6.58 microns.

NMR($CDCl_3$): 0.05 (s, 3H), 0.08 (s, 3H); 0.83 (s, 9H); 1.26 (d, 3H); 1.32 (d, 3H); 3.36-4.47 (c, 5H); 5.24 (q, 2H); 5.58 (d, 1H); 6.86 (m, 1H); 6.97 (m, 1H); 7.4 (m, 1H); 7.5 (d, 2H); and 8.1 (d, 2H) ppm.

PREPARATION N

(2-Methylcarbonylthio)propyl)imidazole

A solution of 500 mg. (1.8 mmole) (2-(p-methylphenylsulfonyloxy)propyl)imidazole and 1.14 g (3.6 mmole) tetrabutylammonium thioacetate in 30 ml. acetone was refluxed under nitrogen overnight. The reaction mixture was concentrated to dryness in vacuo and the residue was dissolved in 40 ml. ethyl acetate. The ethyl acetate solution was washed with 40 ml. water, 40 ml. saturated aqueous sodium chloride solution, 40 ml. water and 40 ml. saturated sodium chloride solution, dried over anhydrous sodium sulfate and concentrated in vacuo. The crude product was purified by column chromatography on silica gel (80 g), eluting with 95:5 ethyl acetate-methanol to yield 160 mg of the title compound (48% yield).

NMR ($CDCl_3$): 1.22 (d, 3H); 2.32 (s, 3H); 3.56-4.27 (c, 3H); 6.95 (m, 1H); 7.0 (m, 1H); and 7.44 (m, 1H) ppm.

## PREPARATION O

## (2-(p-Methylphenylsulfonyloxy)propyl)imidazole

p-Toluenesulfonyl chloride (1.9 g, 0.01 mole) was added portionwise to a solution of 1-(1-imidazolyl)-2-propanol (1.26 g, 0.01 mole) and 4-dimethylaminopyridine (2.44 g, 0.02 mole) in 60 ml. methylene chloride which had been cooled to 0°C under nitrogen. The solution was stirred at 0°C for 30 min., then at 25°C overnight. Water (60 ml.) was added and the pH of the resulting mixture was adjusted to 7.0 with 1N aqueous hydrochloric acid solution. The methylene chloride solution was separated, washed with 50 ml. saturated aqueous sodium chloride solution, dried over anhydrous sodium sulfate and concentrated to dryness *in vacuo*. The crude product was purified by column chromatography on silica gel (200 g.), eluting with 9:1 ethyl acetate-methanol to yield 1.5 g (54% yield) of the title compound.

NMR ($CDCl_3$): 1.27 (d, 3H); 2.42 (s, 3H); 3.97-4.2 (c, 3H); 4.77 (m, 1H); 6.75 (m, 1H); 7.15 (m, 1H); 6.9 (m, 1H); 7.2 (d, 2H); and 7.6 (d, 2H) ppm.

## PREPARATION P

## (2-Hydroxypropyl)imidazole

A mixture of 27.8 g (0.41 mole) imidazole and 41.2 ml (0.48 mole) propylene carbonate in 70 ml. toluene was refluxed under an atmosphere of nitrogen for 5 hours. The reaction mixture was cooled and the lower product layer was separated from the toluene layer and purified by vacuum distillation. 43.5 g (84% yield) of product was obtained as a colorless liquid b.p. 145-148° (1.8 mm.).

0132101

P.C. 6726A

## CLAIMS

1. A compound of the formula

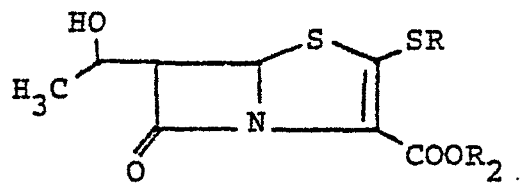

or a pharmaceutically acceptable salt thereof, wherein:

R is

$$-(alk)-X-R_1$$

X is imidazolyl, 1,2,3-triazolyl, 1,2,4-triazolyl or tetrazolyl;

$R_1$ is hydrogen or alkyl having 1-4 carbon atoms;

$R_2$ is hydrogen or a group which forms an ester which is hydrolyzed *in vivo*; and

alk is alkylene having 1-4 carbon atoms.

2. A compound according to claim 1 wherein $R_2$ is hydrogen and R is 1-(imidazol-1-yl)prop-2-yl or (imidazol-4-yl)ethyl.

3. A compound according to claim 1 wherein $R_2$ is hydrogen and R is 2-(imidazol-1-yl)ethyl.

4. A compound according to claim 1 wherein $R_2$ is hydrogen and R is (1-methylimidazol-2-yl)methyl.

5. A pharmaceutical composition comprising a compound according to claim 1 and a pharmaceutically acceptable diluent or carrier.

6. A compound of the formula

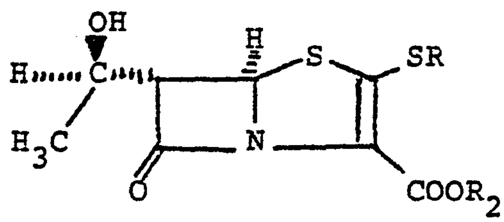

or a pharmaceutically acceptable salt thereof, wherein:

R is

$$-(alk)-X-R_1$$

X is imidazolyl, 1,2,3-triazolyl, 1,2,4-triazolyl or tetrazolyl;

$R_1$ is hydrogen or alkyl having 1-4 carbon atoms;

$R_2$ is hydrogen or a group which forms an ester which is hydrolyzed in vivo; and

alk is alkylene having 1-4 carbon atoms.

7. A compound according to claim 6 wherein $R_2$ is hydrogen and R is 1-(imidazol-1-yl)prop-2-yl or (imidazol-4-yl)methyl.

8. A compound according to claim 6 wherein $R_2$ is hydrogen and R is 2-(imidazol-1-yl)ethyl.

9. A compound according to claim 6 wherein $R_2$ is hydrogen and R is (1-methylimidazol-2-yl)methyl.

10. A pharmaceutical composition comprising a compound according to claim 6 and a pharmaceutically acceptable diluent or carrier.

CLAIMS for the Contracting state : AT

1. A process for preparing a compound of the formula

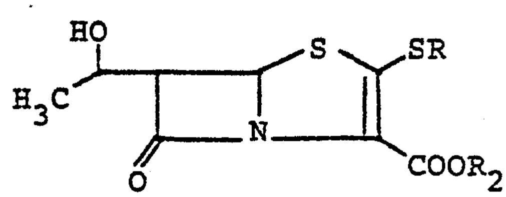

---I

or a pharmaceutically acceptable salt thereof, wherein:

R is

$$-(alk)-X-R_1$$

X is imidazolyl, 1,2,3-triazolyl, 1,2,4-triazolyl or tetrazolyl;

$R_1$ is hydrogen or alkyl having 1-4 carbon atoms;

$R_2$ is hydrogen or a group which forms an ester which is hydrolyzed in vivo; and

alk is alkylene having 1-4 carbon atoms;

characterized by the step of hydrogenating a compound of formula I wherein $R_2$ is a carboxylic acid protecting group.

2. A process according to claim 1 wherein $R_2$ is hydrogen and R is 1-(imidazol-1-yl)prop-2-yl or (imidazol-4-yl)methyl.

3. A process according to claim 1 wherein $R_2$ is hydrogen and R is 2-(imidazol-1-yl)ethyl.

4. A process according to claim 1 wherein $R_2$ is hydrogen and R is (1-methylimidazol-2-yl)methyl.

5. A process for preparing a compound of the formula

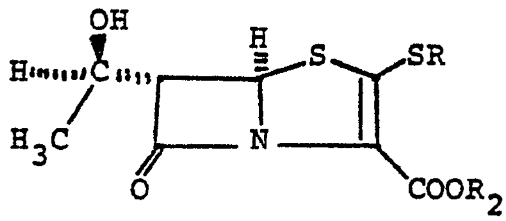

---II

or a pharmaceutically acceptable salt thereof, wherein:

R is

$$-(alk)-X-R_1$$

X is imidazolyl, 1,2,3-triazolyl, 1,2,4-triazolyl or tetrazolyl;

$R_1$ is hydrogen or alkyl having 1-4 carbon atoms;

$R_2$ is hydrogen or a group which forms an ester which is hydrolyzed in vivo; and

alk is alkylene having 1-4 carbon atoms;

characterized by the step of hydrogenating a compound of formula II wherein $R_2$ is a carboxylic acid protecting group.

6. A process according to claim 5 wherein $R_2$ is hydrogen and R is 1-(imidazol-1-yl)prop-2-yl or (imidazol-4-yl)methyl.

7. A process according to claim 5 wherein $R_2$ is hydrogen and R is 2-(imidazol-1-yl)ethyl.

8. A process according to claim 5 wherein $R_2$ is hydrogen and R is (1-methylimidazol-2-yl)methyl.

European Patent Office

**EUROPEAN SEARCH REPORT**

**0132101**
Application number

EP 84 30 4690

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 002 210 (MERCK)<br>* Pages 134-135, compound no. 14 and claims 1-6 *<br>--- | 1,10 | C 07 D 499/00<br>A 61 K 31/43 //<br>C 07 F 7/18<br>C 07 D 233/54 |
| Y | EP-A-0 013 662 (SCHERING)<br>* Page 24, lines 21-22; claims *<br>--- | 1,10 | |
| P,Y | EP-A-0 087 792 (MERCK)<br>* Page 88, formula; page 91, compounds 26,27; claims *<br>----- | 1,10 | |
| | | | TECHNICAL FIELDS SEARCHED (Int. Cl. 3) |
| | | | C 07 D 499/00<br>C 07 F 7/00<br>A 61 K 31/00 |

The present search report has been drawn up for all claims

| Place of search | Date of completion of the search | Examiner |
|---|---|---|
| THE HAGUE | 27-08-1984 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document